# FASCICULE DE BREVET EUROPEEN

(11) **EP 1 330 537 B1**
(45) Date de publication et mention de la délivrance du brevet: **02.05.2007**
(21) Numéro de dépôt: 01982564.5
(22) Date de dépôt: 26.10.2001
(51) Int. Cl.: C12Q 1/48, C11B 1/02, C12N 11/16, C12Q 1/44

(54) **Fractions membranaires de cellules enrichies en 1,2-sn-diacylglycérol**
Membranfraktionen von mit 1,2-sn-Diacylglycerol angereicherten Zellen
Membrane fractions of cells enriched in 1,2-sn-diacylglycerol

(30) Priorité: 31.10.2000 FR 0013976
(43) Date de publication de la demande: 30.07.2003
(73) Titulaire: COMMISSARIAT A L'ENERGIE ATOMIQUE, 75752 Paris (FR); CENTRE NATIONAL DE LA RECHERCHE SCIENTIFIQUE, 75016 Paris Cédex (FR)
(72) Inventeur: MARECHAL, Eric, F-38000 GRENOBLE (FR); MIRAS, Stéphane, F-38190 LANCEY (FR); JOYARD, Jacques, F-38240 MEYLAN (FR)
(74) Mandataire: Goulard, Sophie
(86) Numéro de dépôt international: PCT/FR2001/003329
(87) Numéro de publication internationale: WO 2002/036811

(56) Documents cités:
- FR-A- 2 790 915
- DORNE A-J ET AL: "DO THYLAKOIDS REALLY CONTAIN PHOSPHATIDYLCHOLINE?" PROCEEDINGS OF THE NATIONAL ACADEMY OF SCIENCES OF THE UNITED STATES, vol. 87, no. 1, 1990, pages 71-74, XP002178790 1990 ISSN: 0027-8424
- MIEGE CHRISTINE ET AL: "1,2-sn-Diacylglycerol in plant cells: Product, substrate and regulator." PLANT PHYSIOLOGY AND BIOCHEMISTRY (PARIS), vol. 37, no. 11, novembre 1999 (1999-11), pages 795-808, XP001025688 ISSN: 0981-9428
- MIÈGE, C: "Caractérisation moléculaire et biochimique de la Monogalactosyl Diacylglycerol Synthase (MGDG Synthase) de l'enveloppe des chloroplastes d'épinard" 19 février 1998 (1998-02-19) , UNIVERSIT¹ JOSEPH FOURIER , GRENOBLE XP002178791 page 130, ligne 32 -page 132, ligne 7; tableaux 10,11 page 137, ligne 15 - ligne 42
- MAR¹CHAL E ET AL: "Kinetic Properties of Monogalactosyldiacylglycerol Synthase from Spinach Chloroplast Envelope Membranes" JOURNAL OF BIOLOGICAL CHEMISTRY, AMERICAN SOCIETY OF BIOLOGICAL CHEMISTS, BALTIMORE, MD, US, vol. 269, no. 8, 25 février 1994 (1994-02-25), pages 5788-5798, XP002123832 ISSN: 0021-9258
- SHIMOJIMA M ET AL: "Cloning of the gene for monogalactosyldiacylglycerol synthase and its evolutionary origin" PROCEEDINGS OF THE NATIONAL ACADEMY OF SCIENCES OF USA, NATIONAL ACADEMY OF SCIENCE. WASHINGTON, US, vol. 94, janvier 1997 (1997-01), pages 333-337, XP002123833 ISSN: 0027-8424

## Description

La présente invention est relative à des fractions membranaires de cellules contenant une monogalactosyldiacylglycérol (MGDG) synthase recombinante et enrichies en 1,2-*sn*-diacylglycérol (DAG), à leur procédé de préparation, leur utilisation pour le criblage de molécules effectrices de l'activité de la MGDG synthase ainsi qu'à un procédé de criblage de molécules effectrices de l'activité de la MGDG synthase mettant en oeuvre ces fractions membranaires.

Le MGDG est connu dans tous les plastes analysés à ce jour : il est le lipide le plus abondant des membranes plastidiales où il représente plus de 50% des glycérolipides. Le MGDG est vital à la biogénèse des plastes et à la survie des cellules, et n'existe pas dans les autres systèmes membranaires, en particulier dans les cellules animales (Douce, Sciences, 1974, 183, 852-853) ; sa biosynthèse est catalysée dans l'enveloppe par une uridine 5'-diphosphate-galactose (UDP-gal) 1,2-diacylglycérol 3-β-D-galactosyl-transférase (EC 2.4.1.46) appelée aussi MGDG synthase, selon la réaction suivante :

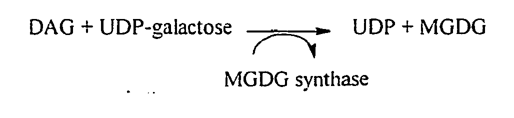
DAG + UDP-galactose UDP + MGDG
MGDG synthase

La MGDG synthase est une enzyme bifonctionnelle à fixation non ordonnée des substrats (Maréchal *et al.,* J. Biol. Chem., 1994, **269,** 5788-5798), possédant des cystéines sensibles à l'oxydation et importante pour la catalyse (Maréchal *et al.,* J. Biol. Chem., 1995, **270,** 11, 5714-5722).

La MGDG synthase est donc également une enzyme capitale à la biogénèse des plastes et est, par conséquent, une cible de choix pour la sélection ou le criblage de molécules à potentiel herbicide. De plus, les parasites responsables du paludisme (4 espèces de *Plasmodium* dont le *P. falciparum),* de la toxoplasmose (*Toxoplasma gondii*) et de fléaux vétérinaires tels que la coccidiose (*Eimeria*) contiennent des chloroplastes dégénérés (apicoplastes) dont il a été démontré qu'ils étaient essentiels à la survie des parasites (G. Mc Fadden and David Roos, "Apicomplexan plastids as drug targets", 1999, 7, n°8, 328-333).

Les parasites contenant ces apicoplastes sont appelés parasites apicomplexes.

Une molécule ayant une action inhibitrice de l'activité de la MGDG synthase possède donc un haut potentiel herbicide et anti-parasitaire apicomplexe et peut être utilisée de façon avantageuse comme nouveau médicament efficace contre lesdits parasites apicomplexes ou comme herbicide.

L'utilisation d'une MGDG synthase pour la sélection ou le criblage de produits inhibiteurs de l'activité de la MGDG synthases, aptes à servir d'herbicides ou de principes actifs contre les parasites apicomplexes, a déjà été proposée, notamment dans la demande de brevet FR-A-2 790 915.

Selon cette demande, la sélection et/ou le criblage de tels produits sont effectués selon un procédé comprenant l'incubation d'une substance à tester avec une MGDG synthase incluse dans des membranes biologiques, puis la mesure de l'activité spécifique enzymatique, après ladite incubation.

Les membranes biologiques utilisées dans cette demande antérieure peuvent notamment être des membranes plastidiales isolées de plantes ou bien encore des fractions membranaires d'E. *coli* surexprimant une MGDG synthase recombinante.

La mesure de l'activité de galactosylation effectuée par la MGDG synthase est à ce jour très complexe et ne permet pas une miniaturisation facile en particulier pour les raisons suivantes.
(1) Addition de deux substrats : Les deux substrats de l'enzyme (DAG et UDP-gal) doivent être ajoutés simultanément dans le milieu d'incubation. Un problème d'homogénéité du système se pose, en particulier du fait que ces deux substrats ont des propriétés physico-chimiques très différentes : l'un est très hydrophile (l'UDP-gal), l'autre est très hydrophobe (le DAG). Le contrôle de l'apport de ces deux substrats est donc difficile à miniaturiser.
(2) Addition d'un détergent : Le DAG est si hydrophobe qu'il n'est pas miscible à l'eau. Pour que l'enzyme ait accès à ce substrat exogène, il est donc obligatoire d'introduire un détergent dans le milieu d'incubation, par exemple le 3-[(3-cholamidopropyl)-diméthylammonio]-1-propanesulfonate (CHAPS), qui pulvérise les membranes biologiques et permet un ré-agencement de l'ensemble des molécules hydrophobes sous forme de micelles. Les micelles sont trop petites pour être séparées du milieu réactionnel par une filtration ou une centrifugation (rayon de Stoke 3,8 nm ; Maréchal *et al.,* 1994, pré-cité).
(3) Séparation de phases : pour extraire la phase lipidique contenant le produit de la réaction (MGDG), un mélange de solvants organiques (chloroforme et méthanol) est ajouté en fin de réaction selon la méthode décrite par Bligh *et al.* (Can. J. Biochem. Physiol., 1959, **37**, 911-917). Un biphase se forme et les lipides sont récupérés dans la phase organique inférieure. La formation d'un biphase et l'extraction d'une phase organique sont des procédés trop sophistiqués pour servir dans un processus miniaturisé.

Or, dans le cadre de la recherche de molécules effectrices sur l'activité de la MGDG synthase, il est indispensable de pouvoir disposer d'une méthode simple, peu coûteuse, rapide et miniaturisable, permettant de tester un très grand nombre de molécules potentiellement aptes à servir d'herbicides ou de principes actifs contre les parasites apicomplexes.

C'est afin de remédier à ces problèmes que les Inventeurs ont mis au point ce qui fait l'objet de l'invention.

Les Inventeurs ont en effet mis au point de nouvelles fractions de membranes biologiques contenant, dans le même feuillet lipidique, à la fois la MGDG synthase et le DAG. Ces membranes peuvent être utilisées dans un procédé enzymatique permettant le criblage et la sélection automatisés à haut débit (High Throughput Screening : HTS) de molécules effectrices sur l'activité de la MGDG synthase (inhibiteurs ou activateurs).

La présente invention a donc pour objet des fractions de membranes plasmiques de cellules procaryotes ou animales eucaryotes renfermant au moins une MGDG synthase recombinante, caractérisées en ce que lesdites fractions contiennent au moins 1% en poids de DAG par rapport au poids total protéique et qu'elles se présentent sous la forme de vésicules sphériques formées d'une bicouche lipidique.

Les Inventeurs ont en effet démontré que la présence simultanée de MGDG synthase et de DAG au sein desdites fractions membranaires permet d'utiliser ces fractions membranaires dans un procédé de criblage et/ou de sélection de molécules effectrices sur l'activité de la MGDG synthase, dans lequel les volumes des milieux réactionnels peuvent être considérablement réduits, permettant ainsi la miniaturisation dudit procédé.

Grâce à ces fractions membranaires, l'ajout de détergent pour favoriser le mélange contrôle MGDG synthase / DAG est supprimé, la réaction enzymatique intervient directement à l'intérieur des membranes.

Selon une forme de réalisation préférée de l'invention, le rapport molaire MGDG synthase / DAG est inférieur à 10, et encore plus particulièrement inférieur à 0,12.

En effet, le ratio enzyme/substrat doit respecter les conditions de mesure permettant d'utiliser le modèle enzymologique de Michaëlis et Menten, en particulier, le substrat ne doit pas être le facteur limitant de la réaction initiale.

Les fractions membranaires conformes à l'invention se présentent sous la forme de vésicules sphériques formées d'une bicouche lipidique dont la taille est généralement comprise entre 0,1 µm et 10 µm.

Au sein de ces vésicules, la MGDG synthase est, en général, localisée sur la face interne de la bicouche lipidique.

Ces vésicules peuvent se présenter sous la forme de vésicules non inversées, inversées ou hybrides.

En effet, la disruption des membranes permet la formation de vésicules inversées et non inversées, mais dans les deux cas, la MGDG synthase est sur la face opposée au plus grand nombre de DAG. Une fusion entre vésicules inversées et non inversées, peut générer une nouvelle famille de vésicules ayant dans le même feuillet lipidique la MGDG synthase et le DAG.

Ce type de vésicules hybrides peut présenter une activité catalytique accrue (substrat accessible) et est par conséquent préféré selon l'invention. L'invention a également pour objet un procédé de préparation de fractions membranaires telles que décrites précédemment, caractérisé par le fait qu'il consiste :
- dans une deuxième étape, à cultiver lesdites cellules dans un milieu de culture favorable à la synthèse protéique, de façon à induire la synthèse de MGDG synthase par lesdites cellules,
- dans une troisième étape, à incuber les cellules cultivées à l'étape précédente dans un milieu réactionnel contenant au moins une phospholipase C,
- puis dans une quatrième étape, à fractionner les cellules ainsi enrichies en DAG pour obtenir des fractions membranaires sous forme de vésicules sphériques formées d'une bicouche lipidique renfermant au moins une MGDG synthase recombinante et au moins 1% en poids de DAG par rapport au poids total en protéines desdites fractions membranaire.

Parmi les cellules procaryotes pouvant être utilisées selon ce procédé, on peut citer les bactéries telles que *E. coli,* qui sont particulièrement préférées selon l'invention.

Parmi les cellules eucaryotes pouvant être utilisées selon ce procédé, on peut citer les cellules de levures telles que *Saccharomyces cerevisiae,* les cellules d'insectes tels que les drosophiles ainsi que les cellules de mammifères habituellement utilisées pour l'expression de gènes telles que les cellules COS et les cellules CHO.

La transformation des cellules lors de la première étape est de préférence réalisée sur des cellules bactériennes et plus particulièrement sur des cellules d'*E. coli.*

Cette transformation peut être réalisée selon la méthode décrite dans la demande de brevet FR-A-2 790 915, par exemple par choc thermique avec un plasmide pET-Y3a contenant la séquence codant pour la MGDG synthase A *d'Arabidopsis thaliana* (Miège *et al.,* Eur. J. Biochem., 1999, **265,** 990-1001).

Le milieu de culture utilisé lors de la deuxième étape est un milieu de culture riche, afin de favoriser l'expression de la MGDG synthase, et est choisi en fonction du type de cellules à cultiver. Dans le cas particulier des cellules bactériennes, et notamment de *E. coli,* on peut utiliser le milieu Luriat Broth (LB).

L'utilisation de PLC au cours de la troisième étape du procédé de préparation conforme à l'invention est indispensable à l'enrichissement des membranes cellulaires plasmiques en DAG.

En effet, ces membranes cellulaires sont riches en phospholipides, particulièrement en phosphatidyléthanolamine (80% des phospholipides). Ces phospholipides peuvent être hydrolysés par la PLC qui n'est pas spécifique de la tête polaire.

La PLC catalyse la transformation de la phosphatidyléthanolamine en DAG selon la réaction suivante :

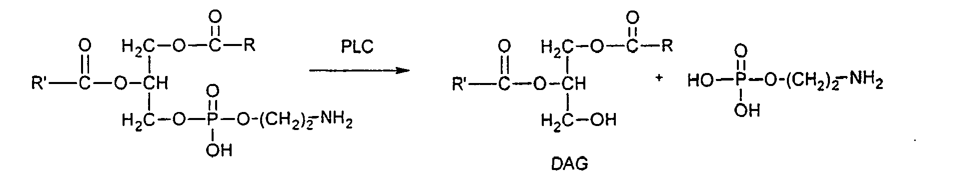

La PLC permet donc d'enrichir les membranes cellulaires en DAG par l'hydrolyse de ses phospholipides.

La nature de la PLC utilisée selon l'invention n'est pas critique, à condition qu'elle soit active sur les phospholipides des membranes que l'on souhaite enrichir en DAG.

Selon une forme de réalisation particulièrement préférée de l'invention, on utilise une phospholipase C de *Bacillus cereus.*

La PLC est de préférence utilisée à une concentration comprise entre 1 U et 20 U par ml de milieu réactionnel, et encore plus préférentiellement à une concentration comprise entre 5 et 12 U/ml.

Bien entendu, le milieu réactionnel utilisé lors de la troisième étape est en général un milieu tampon dont le pH doit être compatible avec le bon fonctionnement de la PLC. Ce pH est en général compris entre 6 et 8.

Lors de la quatrième étape, les membranes sont fractionnées pour générer des fractions membranaires qui se referment spontanément sous la forme de vésicules membranaires. Le fractionnement des membranes peut être effectué par choc mécanique par exemple au moyen d'une presse de French, par choc thermique (congélation/décongélation), par choc osmotique, électrique ou bien encore physique tel que par sonication.

Les fractions membranaires ainsi obtenues peuvent ensuite éventuellement être purifiées par exemple sur coussin de Percoll.

Lorsque la préparation des fractions membranaires conformes à l'invention est terminée, celles-ci peuvent éventuellement être congelées avant d'être utilisées pour la sélection ou le criblage de molécules effectrices de l'activité de la MGDG synthase.

L'invention a donc également pour objet l'utilisation des fractions membranaires telles que décrites ci-dessus pour la sélection et/ou le criblage *in vitro* de molécules effectrices de l'activité de la MGDG synthase.

En particulier, l'invention a pour objet l'utilisation des fractions membranaires telles que décrites ci-dessus pour la sélection ou le criblage *in vitro* de molécules inhibitrices de l'activité de la MGDG synthase, aptes à servir de principes actifs contre les parasites ou d'herbicides.

L'invention a également pour objet un procédé de sélection et/ou de criblage de molécules effectrices de l'activité de la MGDG synthase, caractérisé en ce qu'il comprend :
- une étape d'incubation de la ou des substances à tester avec une quantité suffisante de fractions membranaires telles que définies précédemment et d'UDP-galactose radiomarqué dans une phase aqueuse ayant un pH compris entre 4 et 11,
- une étape de lavage(s) des fractions membranaires,
- une étape de séparation des fractions membranaires,
- puis une étape de détermination de l'activité de la MGDG synthase.

Selon ce procédé, la réaction enzymatique a lieu directement à l'intérieur des fractions membranaires dont la taille est compatible avec une séparation du milieu réactionnel par centrifugation ou par filtration. Par ce moyen, il n'est plus nécessaire d'utiliser de solvant organique pour extraire les produits de la réaction, le MGDG étant en effet piégé dans les fractions membranaires sur lesquelles une simple mesure de la radioactivité du galactose radiomarqué incorporé peut être effectuée.

Selon une forme de réalisation préférée de l'invention, la phase aqueuse d'incubation contient un tampon et présente un pH compris entre 6 et 8.

A titre d'exemple, le tampon peut notamment être de l'acide 3-(N-moipholino)-propane sulfonique ou un mélange KCl/K₂HPO₄.

La quantité d'UDP-galactose à utiliser dans le milieu d'incubation doit bien entendue être suffisante pour ne pas constituer un facteur limitant de la réaction. Cette quantité est de préférence comprise entre 0,1 et 10 nmoles par µl de milieu réactionnel.

L'étape d'incubation est de préférence conduite à température ambiante, pendant une durée d'au moins 10 secondes et encore plus particulièrement pendant une durée comprise entre 1 et 45 minutes.

A la fin de l'étape d'incubation, la réaction est de préférence arrêtée par refroidissement du milieu d'incubation (en général à une température d'environ 4°C) ou par centrifugation.

Lorsque la réaction est terminée, l'étape de lavage des fractions membranaires est réalisée afin d'éliminer l'excès d'UDP-galactose radiomarqué n'ayant pas été incorporé par les fractions membranaires. Un ou plusieurs lavages successifs peuvent être effectués, en général avec de l'eau.

Les fractions membranaires sont ensuite séparées par centrifugation ou par filtration, cette dernière technique étant particulièrement adaptée à la miniaturisation du procédé sur des microplaques.

La détermination de l'activité de la MGDG synthase est effectuée par la mesure de la quantité de galactose radiomarqué incorporé dans les fractions membranaires. Cette mesure est effectuée de façon classique au moyen d'un compteur de radioactivité.

Le procédé de sélection et/ou de criblage de criblage de molécules effectrices de l'activité de la MGDG synthase conforme à l'invention est facilement miniaturisable car il met en oeuvre de faibles volumes réactionnels et ne fait pas appel à l'utilisation de détergents, ni de solvant organique comme c'est le cas dans les procédés précédemment décrits dans l'art antérieur.

L'invention a donc également pour objet des plaques de microtitration comportant une multitude de puits caractérisées en ce que le fond des puits est constitué d'un filtre et que lesdits puits renferment des fractions membranaires telles que décrites précédemment.

Ces microplaques peuvent être conservées au congélateur avant utilisation. Elles peuvent éventuellement être munies d'un fond amovible.

Les microplaques conforment à l'invention permettent d'effectuer la détermination de l'activité de la MGDG synthase par mesure directe de la radioactivité du galactose radiomarqué incorporé dans les fractions membranaires retenues par le filtre au fond des puits.

Elles peuvent donc être utilisées pour tester d'importantes chimiothèques de molécules vis-à-vis de leur activité effectrice sur l'activité de la MGDG synthase.

Enfin, l'invention a pour objet l'utilisation d'au moins une molécule inhibitrice de l'activité de la MGDG synthase telle que sélectionnée conformément au procédé de criblage et de sélection conforme à l'invention, pour la préparation d'un médicament anti-parasitaire ou d'un herbicide.

Outre les dispositions qui précèdent, la description se réfère à un exemple de préparation de fractions membranaires conformes à l'invention, à un exemple comparatif de détermination de l'activité de synthèse du MGDG, à un exemple de démonstration de la présence de MGDG chez un parasite apicomplexe, ainsi qu'aux figures annexées, dans lesquelles :
- la figure 1 représente le nombre de nmole de galactose incorporé par heure dans des fractions membranaires enrichies en DAG en fonction du nombre de µg de protéines,
- la figure 2A représente le nombre de nmole de galactose incorporé par heure et par mg de protéines (fractions membranaires enrichies en DAG) en fonction du nombre de µmole de DAG,
- la figure 2 B représente une courbe en coordonnées inverses de type Line Weaver et Burk sur laquelle l'inverse du nombre de µmoles de galactose incorporé par heure et par mg de protéines est exprimé en fonction de l'inverse du nombre de µmoles de DAG ;
- la figure 3 représente la mesure de la quantité de galactose marqué incorporée par les lipides membranaires de *Toxoplasma gondii.*

Il doit être bien entendu toutefois que ces exemples sont donnés uniquement à titre d'illustration de l'objet de l'invention, dont ils ne constituent en aucune manière une limitation.

### EXEMPLE 1 : PRÉPARATION DE FRACTIONS MEMBRANAIRES RENFERMANT UNE MGDG SYNTHASE ET DU DAG

### 1) Production de MGDG synthase recombinante chez E. coli

### A - Transformation des bactéries

Les transformations des bactéries ont été effectuées selon la méthode décrite dans la demande de brevet FR-A-2 790 915.

Toutes les cultures sont réalisées en conditions stériles. Des bactéries compétentes (souche bactérienne BL21 ou BLR *d'Escherichia coli)* sont transformées par choc thermique avec un plasmide pET-Y3a qui permet de surmonter le problème dû au fait que la séquence déduite de la MGDG synthase contient 22 résidus arginine, parmi lesquels 17 sont codés par AGG ou AGA, codons qui sont en fait peu utilisés chez *E. coli.*

Le plasmide pET-Y3a a été décrit dans la demande de brevet FR-A-2 790 915 et est construit en insérant dans le plasmide pET-3a (Novagen), le gène arg U (ou DNA Y) codant pour l'ARN de transfert de l'arginine associé aux codons rares AGA/AGG. Le plasmide pET-Y3a contient la séquence codant pour la MGDG synthase A *d'Arabidopsis thaliana* (sous contrôle d'un promoteur inductible par l'isopropyl-β-D-thiogalactopyranoside : IPTG), un gène de résistance à la Carbenecilline et une séquence codant pour l'ARN de transfert arginine. Cet ARNt *ARG4* permet la synthèse de protéines telles que la MGDG synthase dont la séquence contient de nombreux codons Arg, rares dans la bactérie.

### B - Production de MGDG synthase A recombinante chez E. coli

Une colonie isolée de bactéries recombinantes est transférée dans 8 ml de milieu Luriat Broth (LB) en présence d'antibiotique (20 µg/ml final de Carbenecilline). La pré-culture est incubée à 37°C, sous agitation régulière et l'évolution de la croissance bactérienne est suivie par mesure de la densité optique (DO) à 600 nm, jusqu'à obtention d'une valeur de 0,5.

La pré-culture est transférée dans 500 ml de milieu LB (20 µg/ml final de Carbenecilline) et incubée à 37°C sous agitation régulière. A une DO mesurée à 600 nm de 0,5, la population bactérienne est alors dans sa phase exponentielle de croissance qui est un moment de synthèse protéique intense.

L'ajout d'IPTG (0,4 mM final) permet d'induire la synthèse de la MGDG synthase recombinante.

La culture est ensuite incubée 3 heures sous agitation à 28°C pour favoriser la production de la protéine sous sa forme active.

La suspension de bactéries induites est répartie dans deux fractions de 250 ml et soumises à une centrifugation de 15 minutes à 5000 tours/mn (Centrifugeuse Sorvall ® RCSC, rotor GS-3).

Les culots récupérés sont resuspendus dans 10 ml de milieu de culture (LB, Carbenecilline à 20 µg/ml final) et centrifugés pendant 15 minutes à 5000 tours/mn (Sorvall ® RCSC, rotor SLA 600 TC).

Le surnageant est éliminé et le culot bactérien conservé à -80°C.

### 2) Enrichissement des membranes bactériennes en DAG par traitement à la phospholipase C.

5 ml de culot des bactéries induites en MGDG synthase à l'étape précédente sont resuspendus dans 5 ml d'acide 3-(N-morpholino)-propane sulfonique (MOPS) 10 mM, pH 7,8, de dithiothréitol (DTT) 1 mM et de glycérol 10% (p/v).

20 µl de phospholipase C (PLC) de *Bacillus cereus* (phosphatidylcholine cholinephospho-hydrolase, EC 3.1.4.3) sont ajoutés afin d'obtenir une concentration finale de 8 U/ml. La réaction se déroule à température ambiante pendant 3 heures sous agitation et est arrêtée par ajout d'EDTA (0,4 M final).

### 3) Purification des membranes bactériennes

Une solution des bactéries induites, dont les membranes sont enrichies en DAG par traitement à la PLC telles qu'obtenues ci-dessus à l'étape précédente, est reprise dans 50 ml de MOPS 5 mM pH 7,8, DTT 0,5 mM, glycérol 5% (p/v).

Les bactéries sont éclatées à haute pression (800 psi) grâce à une presse de French : les membranes en solution aqueuse s'organisent alors spontanément en vésicules et en vésicules retournées.

6 ml du lysat obtenu sont déposés sur un coussin de Percoll à 35% (dans du MOPS 10 mM, pH 7,8, DTT 1 mM, glycérol 10 %) puis centrifugés pendant 10 minutes à 5000 tours/mn, à une température de 4°C (Sorvall ® RC SC, rotor HB-6).

On obtient des vésicules constituées d'une bicouche lipidique contenant de la MGDG synthase et au moins 1% en poids de DAG par rapport au poids total protéique.

Quatre fractions sont alors formées : un surnageant (dans la partie supérieure du tube), l'interface surnageant / Percoll, le coussin de Percoll 35% et un culot.

Toutes les fractions membranaires sont collectées et lavées par dilution avec 5 volumes de MOPS 10 mM pH 7,8, DTT 1 mM, glycérol 10% (p/v) et centrifugées 15 minutes à 5000 tours/mn à une température de 4°C (Sorvall ® RCSC, rotor HB-6).

Les surnageants sont éliminés et les culots, fragiles, sont repris délicatement dans 1 ml de milieu de lavage pour être à nouveau centrifugés pendant 10 minutes à 13000 tours/mn à une température de 4°C (centrifugeuse Eppendorff centrifuge 5804).

Les culots sont resuspendus dans 500 µl de MOPS 10 mM pH 7,8, DTT 1 mM, glycérol 10 % (p/v) et sont conservées à -20°C.

### EXEMPLE 2 : DÉTERMINATION COMPARATIVE DE L'ACTIVITÉ DE SYNTHÈSE DU MGDG SELON L'ART ANTÉRIEUR ET SELON L'INVENTION

### I - Mesure classique de l'activité de synthèse de MGDG selon la méthode de Bligh et Dver (1959)

Cette méthode a été décrite dans l'article de Bligh et Dyer, "*A Rapid Method Of Total Lipid Extraction and Purification*", Can. J. Biochem. Physiol., 1959, 37, 911-917.

La mesure de l'activité de galactosylation repose sur l'incorporation du galactose provenant de l'UDP-gal radioactif (¹⁴C) dans la fraction lipidique du milieu réactionnel.

La réaction se déroule à température ambiante. 100 µg de substrat hydrophobe DAG (1 mg/ml) ainsi que 200 µg de phosphatidylglycérol (PG) en solution chloroformique (10 mg/ml) sont introduits dans un tube, séchés sous argon puis resuspendus avec 11 µl d'un milieu détersif (CHAPS 85 mM, MOPS à 0,7 M, DTT 14 mM), 75 µl de KCl (1 M) et 75 µl de KH₂PO₄ (1 M).

La présence du détergent, ici le CHAPS, permet de créer des micelles mixtes contenant le détergent, le DAG, le PG et la MGDG synthase provenant de l'échantillon.

Après addition de 150 µl d'échantillon, la composition finale du milieu réactionnel (MOPS 50 mM, DTT 1 mM, KCl 250 mM, KH₂PO₄, 250 mM, CHAPS 6 mM dans 300 µl de volume final) répond aux critères du modèle de dilution de surface (Maréchal *et al,* 1995 pré-cité).

Cet exemple utilise le CHAPS à titre de détergent, mais il est aussi possible d'utiliser d'autres détergents tels que par exemple du cholate, du déoxycholate, du Triton-X100 ®, du NONIDET ®, de l'octylglucoside ou de l'oxyde de lauryldiméthylamine (LDAO).

La réaction est démarrée en introduisant 10 µl d'UDP-[¹⁴C]-galactose (New England Nuclear 25 Bq/µmole, 10 mM). La réaction est stoppée, par addition de 1,5 ml d'un mélange chloroforme/méthanol 1/2 (v/v).

L'addition de 0,5 ml de chloroforme et de 0,6 ml d'eau, permet après centrifugation pendant 10 minutes à 1000 tours/mn (centrifugeuse Eppendorff A-4-44, rotor 5804), d'obtenir une biphase distincte (une phase aqueuse fortement radioactive dans la partie supérieure du tube, et une phase organique dans la partie inférieure).

La phase aqueuse contient la radioactivité de l'UDP-gal résiduel non utilisé par l'enzyme alors que la phase organique contient la radioactivité du produit hydrophobe de la réaction : le MGDG.

Après deux lavages de la phase aqueuse avec un volume identique de phase aqueuse sans UDP-[¹⁴C]-gal, la phase organique contenant le MGDG produit est transférée dans un pot de comptage.

La fraction récupérée est séchée sous argon, reprise dans 10 ml de liquide de scintillation, et la radioactivité de l'échantillon est estimée.

L'activité de la galactosylation est définie par le nombre de µmoles de galactose incorporées dans la fraction lipidique par mg de protéine et par heure.

### II - Mesure par purification des membranes après centrifugation conformément à l'invention.

Cette mesure ne peut être réalisée que pour un échantillon constitué de vésicules de membranes séquestrant à la fois l'enzyme (la MGDG synthase) et son substrat hydrophobe (le DAG). Alors seulement, il n'est plus nécessaire d'ajouter un détergent pour qu'enzyme et substrat entrent en contact.

Le milieu réactionnel utilisé est différent de celui présenté dans la méthode classique après extraction des lipides par les solvants organiques.

L'échantillon de fraction membranaires contenant la MGDG synthase et le DAG, telles qu'obtenues ci-dessus à l'exemple 1, étape 3, est mis en suspension de 300 µl final contenant du KCl 250 mM et du KH₂PO₄ 250 mM.

La réaction est initiée par addition d'UDP-gal radiomarqué [¹⁴C].

La réaction est stoppée par transfert dans la glace pendant 10 minute.

Le lavage de l'UDP-[¹⁴C]gal en excès (non incorporé dans les membranes) se fait par centrifugation de l'échantillon pendant 10 minutes à 13000 tours/mn à une température de 4°C et reprise du culot dans 500 µl d'eau stérile.

Cette étape de lavage est répétée trois fois. Le culot obtenu est séché par centrifugation sous vide pendant 1 heure (Speed-vac Eppendorff Concentrator 5301). L'échantillon sec est ensuite repris dans un mélange chloroforme/méthanol 1/2 (v/v), afin de le transférer dans un pot de comptage, séché, sous argon, et solubilisé avec 10 ml de liquide de scintillation.

La radioactivité de l'échantillon est estimée à l'aide d'un compteur Kontron ® (Betamatic).

L'activité de galactosylation est définie par le nombre de µmoles de galactose incorporées par mg de protéine et par heure.

### III - Électrophorèse sur gel de polyacrylamide en conditions dénaturantes

Les échantillons à analyser sont suspendus dans le milieu de dépôt (Tris HCl 0,15 M, pH 6,8, glycérol 10 %, SDS 0,02 %, bleu de bromophénol 0,01 %, DTT 0.025 %), puis sont portés pendant 4 minutes à ébullition.

Les solutions d'acrylamide sont préparées dans un tampon Tris 25 mM, (pH 8,3 dans le gel de séparation et pH 6,5 dans le gel de concentration), glycine 0,192 M, sodium dodécyl sulfate (SDS) 0,1%.

L'électrophorèse sur gel de concentration (5 % d'acrylamide) puis sur gel de séparation (12 % d'acrylamide) est conduite à température ambiante dans un tampon Tris 25 mM, glycine 0,192 M (pH 8,3), SDS 0,1 % (p/v) (Laemmli U. K., Nature, 1970, 227, 680-683) sous un voltage constant de 100 V.

La migration est stoppée lorsque le bleu de bromophénol sort du gel.

Les protéines sont alors colorées au bleu de Coomassie (Coomassie brilliant blue 8250 0,5 % (p/v), méthanol 25 %, acide acétique 10 % (v/v)).

La décoloration du gel se fait par bains successifs d'isopropanol 25 %, acide acétique 10 % (v/v).

### IV - Dosage des protéines

Principe : les protéines sont dosées par la méthode de Lowry (Lowry *et al.,* J. Biol. Chem., 1951, **193,** 265-275), par mesure de deux réactions colorées simultanées.

Une première réaction analogue à celle du « biuret » conduit à la formation d'un complexe entre les liaisons peptidiques des protéines (-CO-NH-) et les ions Cu²⁺ en milieu alcalin, et une seconde réaction conduit à la réduction du réactif de Folin-ciocalteu par les phénols des tyrosines. Cette méthode permet de doser des solutions de concentration allant de 2 à 200 mg/ml.

Démarche expérimentale : le volume de l'échantillon à doser est ajusté à 200 µl avec de l'eau stérile puis 1 ml de réactif de dosage préparé extemporanément (50 volumes de C03Na2 2%, NaOH 0,1 N + 1 volume de CuS04, SH20 0,5%, tartrate de sodium 1%).

Après 10 minutes de réaction à 20°C, 100 µl de réactif de Folin-ciocalteu sont ajoutés et la réaction est incubée à 20°C pendant 30 minutes.

La quantité de protéines de l'échantillon à doser est déterminée par comparaison d'absorbance à 750 nm avec une gamme étalon établie avec de la sérum albumine bovine (BSA).

### V - RÉSULTATS

### A - Les bactéries recombinantes doivent être enrichies artificiellement en DAG pour permettre la mesure de l'activité de galactosylation en présence d'UDP- Gal

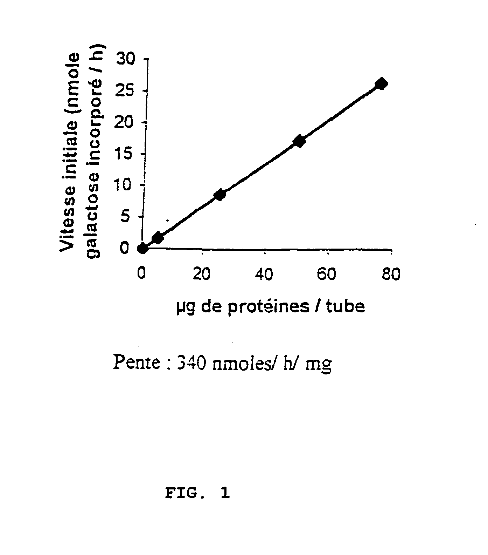

L'analyse des protéines totales d'échantillons de bactéries prélevées au cours de l'étape d'induction (Exemple 1, étape 1, figure 1) montre que l'induction par l'IPTG conduit à une accumulation de MGDG synthase A représentant environ 30% des protéines.

Une comparaison de l'activité de galactosylation mesurée dans un extrait brut de bactéries induites exprimant la MGDG synthase A en présence et en absence de DAG a été réalisée.

La mesure de l'activité de galactosylation à été effectuée par extraction des lipides selon la méthode de Bligh et Dyer (voir ci-dessus) sur 20 µl d'une culture bactérienne induite à 28°C, en absence de PG et mis en présence ou non de 50 µg DAG.

Les résultats obtenus figurent dans le Tableau I ci-après :

**Tableau I**

| **Incubation des bactéries dans 270 µM de DAG** | **Activité de la MGDG synthase (en nmoles de galactose incorporé par heure)** |
|---|---|
| oui | 576 |
| non | 5 |

Ces résultats montrent qu'une activité importante de galactosylation est observée dans cet extrait incubé avec 270 µM de DAG, alors qu'il n'y a pratiquement pas d'incorporation de galactose dans les membranes bactériennes incubées sans apport exogène de DAG.

Par conséquent, les membranes bactériennes ne disposent pas de quantité suffisante de DAG endogène pour réaliser une mesure ponctuelle de l'activité de l'enzyme recombinante.

### B - Un traitement des membranes bactériennes par la PLC génère du DAG disponible pour mesurer une activité de galactosylation

L'activité de galactosylation de la MGDG synthase a été mesurée après synthèse de DAG endogène par la PLC ou après addition de DAG exogène. La mesure de cette activité a été effectuée sur 20 µl d'une même culture bactérienne induite à 28°C, en présence ou non de 3 µl de PLC (2000 U/ml), de 50 µg de DAG (à 1 mg/ml et éventuellement en présence de 1 ou 10 µl de CaCl₂.

Les résultats sont présentés dans le Tableau II ci-après :

**Tableau II**

| **DAG en µM** | **PLC** | **CaCl₂ en µM** | **Galactose incorporé (nmole/heure)** |
|---|---|---|---|
| - | - | - | 7 |
| 270 | - | - | 30 |
| - | + | - | 91 |
| 270 | + | - | 99 |
| 270 | + | 33 | 118 |
| 270 | + | 330 | 109 |
| - | + | 33 | 94 |
| - | + | 330 | 97 |

Ces résultats montrent que le traitement des membranes bactériennes par la PLC permet de mesurer une activité de galactosylation supérieure à celle obtenue après l'ajout de 270 µM de DAG exogène.

La PLC permet donc de charger les membranes des bactéries en DAG par l'hydrolyse de ses phospholipides. La faible incorporation de galactose obtenue lors de l'addition de DAG s'explique par le fait qu'une très faible proportion de DAG ajouté à pu pénétrer dans les membranes bactériennes en l'absence de PLC pour servir de substrat à la MGDG synthase.

Il est également important de remarquer que le calcium qui est un activateur des PLC n'a aucun effet sur l'incorporation de galactose mesurée, suggérant que la concentration en Ca²⁺ de la suspension bactérienne suffit à mesurer une activité optimale de la PLC.

### C - Incorporation de galactose radioactif dans des membranes enrichies en DAG

L'incorporation de galactose radioactif dans les vésicules membranaires lysées telles que décrites ci-dessus à l'exemple 1, étape B-3) a été mesurée et comparée à l'incorporation de galactose radioactif par des bactéries induites pour exprimer la MGDG synthase A, mais non lysées. L'activité de galactosylation est mesurée sur 150 µl d'échantillon (comme décrit précédemment) en absence ou en présence de 150 µg de PG (à 10 mg/ml) et de 100 µg de DAG exogène (à 1 mg/ml).

Les résultats obtenus sont regroupés dans le Tableau III ci-après :

**Tableau III**

| **Bactéries induites, traitées à la PLC** | **Galactose incorporé (nmole/heure)** | |
|---|---|---|
| | **Sans apport exogène de DAG** | ***+* 550 µM de DAG exogène et 667 µM de PG** |
| **Non lysées** | 2426 | 6622 |
| **Lysées** | 3172 | 8663 |

Ces résultats montrent que l'activité de galactosylation mesurée sur des bactéries lysées sans ajout de DAG ni de PG indique que des vésicules contenant à la fois la MGDG synthase et le DAG ont été formées, et que l'enzyme a conservé son pouvoir catalytique.

Ils montrent également que l'activité des fractions lysées est supérieure à celle des fractions non lysées. Cette observation est cohérente avec une localisation de la MGDG synthase A sur la face interne de la bicouche lipidique des membranes bactériennes. En effet, la PLC n'ayant accès qu'à la couche lipidique externe, la fraction de MGDG synthase localisée sur la face interne ne peut capter qu'une portion des DAG formés ayant subit un déplacement transversal du feuillet externe vers le feuillet interne de la membrane. La disruption des membranes permet la formation de vésicules inversées et non inversées, mais dans les deux cas, la MGDG synthase est sur la face opposée au plus grand nombre de DAG. Une fusion entre vésicules inversées et non inversées, peut générer une nouvelle famille de vésicules ayant dans le même feuillet membranaire l'enzyme et le DAG. Ce type de vésicule peut présenter une activité catalytique accrue (substrat accessible). En supposant que la génération de ces trois populations de vésicules, inversées, non inversées, et hybrides soient équiprobable, alors, l'activité des échantillons lysés est augmentée d'un facteur 1,33 par rapport aux échantillons non lysés, ce qui est observé dans le cas présent.

### D- Mise au point d'un test miniaturisable

### 1) Mise en évidence du rôle de la PLC

Une suspension de bactéries lysées contenant des vésicules de membranes enrichies en MGDG synthase A et éventuellement en DAG par traitement à la PLC est fractionnée sur gradient de Percoll 35% (9 ml) comme décrit précédemment. Les fractions correspondant au surnageant et à l'interface (S + SL : 6 ml) ainsi que celles correspondantes au culot (P : 1 ml) sont collectées. L'activité de galactosylation et les quantités de protéines sont mesurées sur les différentes fractions prélevées (S + SL = 6 ml, P = 1 ml) ainsi que sur la fraction déposée (D de 6 ml).

Les résultats obtenus sont présentés dans le Tableau IV ci-après :

**Tableau IV**

| **Fractions** | | **Protéines en µg/ml** | **Activité totale (en nmole UDP-gal incorporées/ heure)** | **Activité spécifique (nmole UDP-gal incorporées/ heure/mg)** | **Enrichissement** |
|---|---|---|---|---|---|
| **Sans traitement à la PLC** | **D** | 400 | 103 | 43 | 1 |
| | **S + SL** | 136 | 363 | 44 - | 10,3 |
| | **P** | 39 | 3 | 83 | 1,9 |
| **Avec traitement à la PLC** | **D** | 500 | 618 | 206 | 1 |
| | **S + SL** | 880 | 11462 | 2169 | 10,5 |
| | **P** | 70 | 67 | 955 | 4,6 |

Ces résultats montrent que les membranes ayant subi un enrichissement en DAG par traitement à la PLC sont capables de transformer beaucoup plus d'UDP-gal que les membranes non enrichies en DAG, car n'ayant pas subi de traitement à la PLC.

L'analyse des fractions par électrophorèse sur gel d'acrylamide en conditions dénaturantes montre un enrichissement d'un polypeptide correspondant à la MGDG synthase, du surnageant vers le culot. La faible activité de galactosylation dans le culot (Tableau IV) suggère un enrichissement de cette fraction principalement dans des corps d'inclusion (enzyme inactive). Les fractions S + SL contenant la MGDG synthase A associée aux membranes enrichies en DAG ont donc été sélectionnées pour analyser l'activité enzymatique de la MGDG synthase en fonction des critères de validité de l'enzymologie Michaélienne.

### 2) Analyse de l'activité enzymatique de la MGDG synthase

Les résultats sont reportés sur la figure 1 qui représente la vitesse initiale (en nmole de galactose incorporé par heure) en fonction de nombre de µg de protéines / tube. Les vitesses initiales ont été déterminées en mesurant l'incorporation de galactose après 15 et 30 minutes d'incubation de différentes quantités des membranes traitées à la PLC et présentes dans les fractions S + SL traitées (25, 50, 75 µg de protéines).

Cette figure 1 montre que l'incorporation de galactose est une fonction linéaire de la quantité d'échantillon incubé en présence d'UDP-gal. La pente de la courbe permet de déduire une activité de 340 nmoles de galactose incorporées par heure et par mg de protéines.

La quantité de DAG obtenu après traitement par la PLC est indéterminée mais la concentration de ce substrat dans les vésicules de membranes reste constante quelle que soit la quantité de protéines de l'échantillon. A ce titre, la concentration de surface de DAG (en fraction molaire ou en mole de DAG par unité de surface membranaire) et la concentration molaire d'UDP-gal sont constantes. De ce fait, la vitesse initiale de réaction est directement proportionnelle à la quantité de protéines montrant le caractère limitant de la quantité d'enzyme. La relation de linéarité est une condition de validation des conditions d'analyse enzymologique Michaélienne.

### 3) Évaluation de la quantité de DAG généré dans les membranes bactériennes par traitement à la PLC

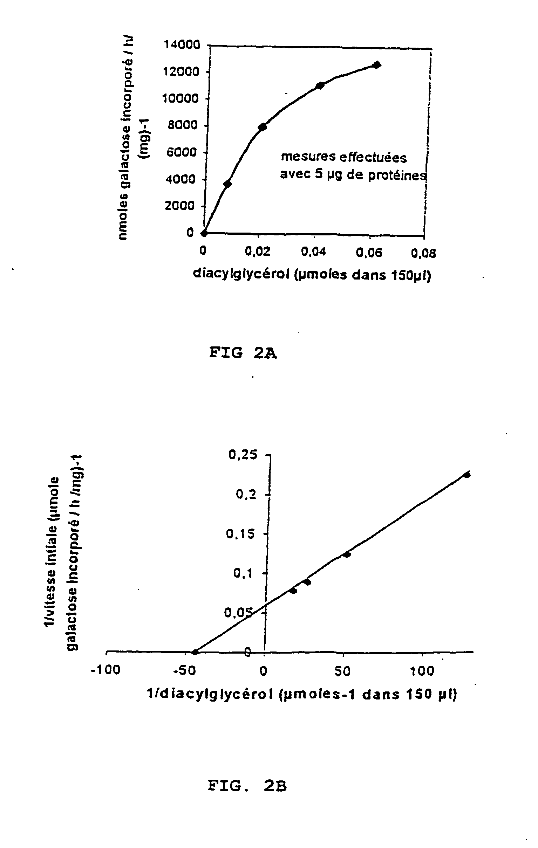

L'activité (en nombre de nmole de galactose incorporé/ heure/mg) de 5 µg de membranes non traitées par la PLC et purifiées sur gradient de Percoll a été mesurée pour des apports variables de DAG (figure 2A). Les vitesses initiales ont été déterminées en mesurant l'incorporation de galactose après 15 et 30 minutes d'incubation. Les mesures ont été réalisées pour des apports variables de DAG (12,5 µg ; 25 µg ; 37,5 µg ; pour 300 µl de milieu réactionnel).

Les mesures permettent d'établir une courbe en coordonnées inverses de type Lineweaver et Burk (figure 2B).

En considérant que les mesures effectuées sur des membranes non-traitées à la PLC constituent des courbes étalon pour estimer une teneur en DAG, un échantillon de 5 µg dont l'activité intrinsèque est de 340 nmoles / h / mg équivaut au même échantillon non traité additionné de 0,5 µg de DAG.

On peut donc en déduire que le traitement à la PLC permet l'obtention de l'ordre de 0,1 µg de DAG par µg de protéines.

### 4) Mesure de l'activité de aalactosylation de membranes enrichies en MGDG synthase et en DAG avant et après décongélation.

La mesure de capacité d'incorporation du galactose par des membranes ayant subi une congélation de un jour à -20°C a été mesurée et comparée à celle des mêmes membranes n'ayant pas subi d'étape de congélation.

Aucune différence significative n'a été observée entre les deux échantillons.

Par conséquent, cette propriété autorise la préparation de l'échantillon de membranes et sa manipulation reproductible dans un automate de criblage de molécules effectrices sur l'activité de la MGDG synthase qui peut comprendre des étapes de conservation de l'échantillon au froid, sans perte d'activité.

### 5) Mesure simplifiée de l'activité de galactosylation contenue dans l'échantillon de membranes enrichies en MGDG svnthase et en DAG

Des vésicules membranaires contenant de la MGDG synthase et enrichies en DAG par traitement par la phospholipase C, éventuellement purifiées peuvent donc être utilisées pour mesurer la production de MGDG dans ces mêmes vésicules.

50 µg de vésicules membranaires (équivalents à 50 µg de MGDG synthase et 50 µg de DAG) sont mis en suspension dans une phase aqueuse (KCl 250 mM, K₂HPO₄ 250 mM, 300 µl final, pH 7,8) contenue dans un tube (Tube n°1).

A titre de comparatif, un tube témoin (tube n°2) a été préparé : 50 µg de MGDG synthase et 50 µg de DAG ont été mis en suspension dans la même phase aqueuse que celle utilisée dans le tube n°1.

La réaction est initialisée dans chaque tube par addition de 10 µl d'UDP-gal radiomarqué au ¹⁴C sur le galactose, puis arrêtée au bout de 30 minutes par refroidissement à 4°C.

Le milieu réactionnel du tube n°1 est ensuite centrifugé à 13000 tours/mn pendant 10 minutes, rincé plusieurs fois avec 500 µl d'eau, puis centrifugé à nouveau à 13000 tours/mn pendant 10 minutes.

Le tube n°2 a été traité par la méthode classique d'extraction des lipides par les solvants organiques, telle que décrite précédemment.

La radioactivité récupérée dans le culot de centrifugation (tube n°1) a été mesurée à l'aide d'un compteur de radioactivité et était de 4026 dpm.

La radioactivité mesurée après extraction des lipides dans le tube n°2 a été mesurée de la même façon et était de 4136 dpm.

Par conséquent, la radioactivité mesurée pour chacun des tubes n'est pas significativement différente et permet de valider le procédé de mesure de l'activité de la MGDG synthase conforme à l'invention.

L'échantillon de vésicules membranaires préparé conformément à l'invention contient l'enzyme et son co-substrat hydrophobe dans des conditions qui répondent à une mesure de l'activité enzymologique par la méthode de Michaelis. De plus, cet échantillon permet une mesure simple et miniaturisable de l'activité de galactosylation par centrifugation, et par extension, par filtration.

### VI - CONCLUSION

Cet exemple démontre qu'il est possible d'utiliser des fractions membranaires dérivées d'une culture de cellules exprimant une MGDG synthase recombinante pour mesurer une activité de galactosylation respectant les lois Michaéliennes et dont la procédure de mesure peut être miniaturisée sur microplaques.

Il a également été démontré que la radioactivité incorporée pouvait être simplement récupérée dans les agrégats du milieu réactionnel collectés par centrifugation. Ainsi, le MGDG généré par ce système s'accumule dans les membranes qu'un système de mesure par microfiltration suffit à mesurer, rendant possible l'utilisation de cet essai enzymatique pour des criblages à haut débit (HTS) de molécules actives. En particulier, il est maintenant possible de cribler une chimiothèque par ce procédé pour sélectionner de nouvelles molécules à potentiel herbicide. Les inhibiteurs spécifiques de la MGDG synthase A ainsi produits seront par ailleurs des outils pharmacologiques puissants pour permettrent les études physiologiques de la synthèse des galactolipides.

### EXEMPLE 3 : MISE EN ÉVIDENCE D'UNE SYNTHÈSE DE MGDG CHEZ UN PARASITE APICOMPLEXE

Cet exemple a pour but de démontrer la présence de MGDG chez parasite apicomplexe, *Toxoplasma gondii* et que par conséquent la MGDG synthase qui sert de cible pour la recherche de molécule à propriétés antiparasitaire existe bien chez les apicomplexes.

2.10⁸ cellules de *Toxoplasma gondii,* sous la forme de tachyzoites, sont suspendues dans 0,1 ml d'un mélange 1:10 d'acide 3-(N-morpholino)-propane sulfonique (MOPS) 10 mM, pH 7,8 et de dithiothréitol (DTT) 1 mM, contenant 2% de glycérol (p/v) et 50 mM de KCl puis incubés pendant 30 mn en présence de 4 µCi d'UDP-[³H]-galactose (7,5 nmole).

Les glycolipides sont extraits selon la méthode de Bligh et Dyer, 1959 (précité), puis analysés par chromatographie sur couche mince (CCM) sur des plaques de gel de silice 60 µ résolues par un mélange chloroforme/méthanol/eau : 65/25/4 (v/v), en présence de lipides témoins (MGDG ; monogalactosylcérébroside de cerveau de boeuf (MGCB) ; digalactosyldiacylglycérol (DGDG) ; trigalactosyldiacylglycérol (triGDG) et tetragalactosyldiacylglycérol (tetra GDG)).

La radioactivité du lipide marqué est ensuite détectée à l'aide d'un appareil TLC-analyser (LB2842 automatic TLC scanner).

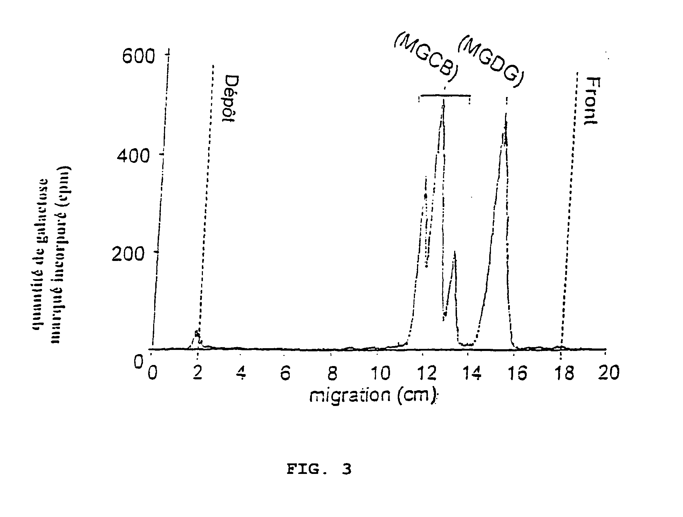

Les résultats obtenus sont reportés sur la figure 3 qui illustre la quantité de galactose marqué (cpm) incorporé par les cellules de *Toxoplasma gondii* en fonction de la migration en centimètres.

On peut voir sur cette figure, 3 premiers pics qui migrent au même niveau que le MGCB, alors que le dernier pic migre au niveau du MGDG.

Après migration, les lipides sont visualisés par pulvérisation, sur les plaques de gel de silice, d'une solution comprenant 0,2 % d'orcinol et 75 % d'acide sulfurique puis chauffage à une température de 100°C pendant 15 minutes (résultats non représentés).

Le pic correspondant au MGDG disparaît après hydrolyse alcaline pendant 3 heures avec de la potasse 0,1 N dans un mélange eau/méthanol.

L'identification complète des lipides est réalisée après hydrolyse de la tête polaire par de l'α-galactosidase de grains de café vert et de la β-galactosidase de testicules bovins et désacylation par hydrolyse alcaline en conditions douces (KOH 0,1 N dans un mélange eau/éthanol pendant 3 heures).

Le pic 4 est sensible à l'hydrolyse à la β-galactosidase, ce qui montre que le galactose est bien lié en position beta comme pour le MGDG.

Par ailleurs, après l'hydrolyse alcaline, le pic 4 disparaît ce qui démontre que le lipide présent dans la membrane du *Toxoplasma gondii* contient bien une moitié de diacylglycérol.

Cette expérience démontre l'existence de MGDG dans la membrane des tachyzoites de *Toxoplasma gondii* et confirme que la recherche d'inhibiteurs de la MGDG synthase de plante a comme application l'identification d'antiparasitaires apicomplexes.

## Revendications

1. Fractions de membranes plasmiques de cellules procaryotes ou animales eucaryotes renfermant au moins une monogalactosyldiacylglycérol (MGDG) synthase recombinante, **caractérisées en ce que** lesdites fractions contiennent au moins 1% en poids de 1,2-sn-diacylglycérol (DAG) par rapport au poids total protéique et qu'elles se présentent sous la forme de vésicules membranaires sphériques formées d'une bicouche lipidique.

2. Fractions selon la revendication 1, **caractérisées par le fait que** le rapport molaire MGDG synthase / DAG est inférieur à 10.

3. Fractions selon la revendication 2, **caractérisées par le fait que** le rapport molaire MGDG synthase / DAG est inférieur à 0,12.

4. Fractions selon l'une quelconque des revendications précédentes, **caractérisées par le fait que** les vésicules membranaires ont une taille comprise entre 0,1 µm et 10 µm.

5. Fractions selon l'une quelconque des revendications précédentes, **caractérisées par le fait que** la MGDG synthase est localisée sur la face interne de la bicouche lipidique.

6. Fractions selon l'une quelconque des revendications précédentes, **caractérisées par le fait que** les vésicules se présentent sous la forme de vésicules non inversées, inversées ou hybrides.

7. Fractions selon la revendication 6, **caractérisées par le fait qu'**elles se présentent sous la forme de vésicules hybrides comportant dans le même feuillet lipidique la MGDG synthase et le DAG.

8. Procédé de préparation de fractions membranaires telles que définies à l'une quelconque des revendications précédentes, **caractérisé par le fait qu'**il consiste :
- dans une première étape, à transformer des cellules procaryotes ou animales eucaryotes par une construction contenant le gène codant pour une MGDG synthase de plante,
- dans une deuxième étape, à cultiver lesdites cellules dans un milieu de culture favorable à la synthèse protéique, de façon à induire la synthèse de MGDG synthase par lesdites cellules,
- dans une troisième étape, à incuber les cellules cultivées à l'étape précédente dans un milieu réactionnel contenant au moins une phospholipase C,
- puis dans une quatrième étape, à fractionner les cellules ainsi enrichies en DAG pour obtenir des fractions membranaires se présentant sous forme de vésicules sphériques formées d'une bicouche lipidique renfermant au moins une MGDG synthase recombinante et au moins 1% en poids de DAG par rapport au poids total en protéines desdites fractions membranaire.

9. Procédé selon la revendication 8, **caractérisé par le fait que** les cellules procaryotes sont des bactéries.

10. Procédé selon la revendication 8 ou 9, **caractérisé par le fait que** l'on utilise une phospholipase C de *Bacillus cereus.*

11. Procédé selon l'une quelconque des revendications 8 à 10, **caractérisé par le fait que** la phospholipase C est utilisée à une concentration comprise entre 1 U et 20 U par ml de milieu réactionnel.

12. Procédé selon l'une quelconque des revendications 8 à 11, **caractérisé par le fait que** le fractionnement des membranes est effectué par choc mécanique, choc thermique, choc osmotique, choc électrique ou par choc physique.

13. Utilisation des fractions membranaires telles que définies à l'une quelconques des revendications 1 à 7, pour la sélection et/ou le criblage *in vitro* de molécules effectrices de l'activité de la MGDG synthase.

14. Utilisation selon la revendication 13, pour la sélection et/ou le criblage *in vitro* de molécules inhibitrices de l'activité de la MGDG synthase, aptes à servir de principes actifs contre les parasites ou d'herbicides.

15. Procédé de sélection et/ou de criblage de molécules effectrices de l'activité de la MGDG synthase, **caractérisé en ce qu'**il comprend :
- une étape d'incubation de la ou des substances à tester avec une quantité suffisante de fractions membranaires telles que définies à l'une quelconque des revendications 1 à 7 et d'UDP-galactose radiomarqué, dans une phase aqueuse ayant un pH compris entre 4 et 11,
- une étape de lavage(s) des fractions membranaires,
- une étape de séparation des fractions membranaires,
- puis une étape de détermination de l'activité de la MGDG synthase.

16. Procédé selon la revendication 15, **caractérisé par le fait que** la phase aqueuse d'incubation contient un tampon et présente un pH compris entre 6 et 8.

17. Procédé selon la revendication 15 ou 16, **caractérisé par le fait que** la quantité d'UDP-galactose est comprise entre 0,1 et 10 nmoles par µl de milieu réactionnel.

18. Procédé selon l'une quelconque des revendications 15 à 17, **caractérisé par le fait que** l'étape de séparation des fractions membranaires est effectuée par centrifugation ou par filtration.

19. Procédé selon l'une quelconque des revendications 15 à 18, **caractérisé par le fait que** la détermination de l'activité de la MGDG synthase est effectuée par la mesure de la quantité de galactose radiomarqué incorporé dans les fractions membranaires.

20. Plaques de microtitration comportant une multitude de puits, **caractérisées en ce que** le fond des puits est constitué d'un filtre et que lesdits puits renferment des fractions membranaires telles que définies à l'une quelconque des revendications 1 à 7.

## Claims

1. Plasma membrane fractions of prokaryotic or eukaryotic animal cells containing at least one recombinant monogalactosyldiacylglycerol (MGDG) synthase, **characterised in that** said fractions contain at least 1% by weight of 1,2-sn-diacylglycerol (DAG), relative to the total weight of protein, and occur in the form of spherical membrane vesicles formed by a lipid bilayer.

2. Fractions according to claim 1, **characterised in that** the molar ratio of MGDG synthase to DAG is less than 10.

3. Fractions according to claim 2, **characterised in that** the molar ratio of MGDG synthase to DAG is less than 0.12.

4. Fractions according to any one of the preceding claims, **characterised in that** the membrane vesicles are between 0.1 µm and 10 µm in size.

5. Fractions according to any one of the preceding claims, **characterised in that** the MGDG synthase is located on the inner surface of the lipid bilayer.

6. Fractions according to any one of the preceding claims, **characterised in that** the vesicles are in the form of non-inverted, inverted or hybrid vesicles.

7. Fractions according to claim 6, **characterised in that** they are in the form of hybrid vesicles containing MGDG synthase and DAG in the same lipid leaflet.

8. Process for preparing membrane fractions as defined in any one of the preceding claims, **characterised in that** it consists:
- in a first step, transforming prokaryotic or eukaryotic animal cells with a construct containing the gene coding for a plant MGDG synthase,
- in a second step, culturing said cells in a culture medium favourable to protein synthesis, so as to induce the synthesis of MGDG synthase by said cells,
- in a third step, incubating the cells cultured in the previous step in a reaction medium containing at least one phospholipase C,
- then in a fourth step, fractionating the cells thus enriched in DAG so as to obtain membrane fractions in the form of spherical vesicles formed by a lipid bilayer containing at least one recombinant MGDG synthase and at least 1% by weight of DAG, relative to the total weight of proteins of said membrane fractions.

9. Process according to claim 8, **characterised in that** the prokaryotic cells are bacteria.

10. Process according to claim 8 or 9, **characterised in that** a phospholipase C from *Bacillus cereus* is used.

11. Process according to any one of claims 8 to 10, **characterised in that** the phospholipase C is used at a concentration of between 1 U and 20 U per ml of reaction medium.

12. Process according to any one of claims 8 to 11, **characterised in that** the fractionation of the membranes is carried out by mechanical shock, thermal shock, osmotic shock, electric shock or physical shock.

13. Use of membrane fractions as defined in any one of claims 1 to 7, for the *in vitro* selection and/or screening of effector molecules for the activity of MGDG synthase.

14. Use according to claim 13, for the *in vitro* selection and/or screening of molecules inhibiting the activity of MGDG synthase, which are suitable for use as anti-parasitic or herbicidal active substances.

15. Process for selecting and/or screening effector molecules for the activity of MGDG synthase, **characterised in that** it comprises:
- a step of incubating the substance or substances to be tested with a sufficient quantity of membrane fractions as defined in any one of claims 1 to 7 and radiolabelled UDP-galactose, in an aqueous phase having a pH of between 4 and 11,
- a step of washing the membrane fractions,
- a step of separating the membrane fractions,
- then a step of determining the activity of the MGDG synthase.

16. Process according to claim 15, **characterised in that** the aqueous incubation phase contains a buffer and has a pH of between 6 and 8.

17. Process according to claim 15 or 16, **characterised in that** the quantity of UDP-galactose is between 0.1 and 10 nmol per µl of reaction medium.

18. Process according to any one of claims 15 to 17, **characterised in that** the step of separating the membrane fractions is carried out by centrifugation or filtration.

19. Process according to any one of claims 15 to 18, **characterised in that** the activity of the MGDG synthase is determined by measuring the quantity of radiolabelled galactose incorporated in the membrane fractions.

20. Microtitre plates comprising a plurality of wells, **characterised in that** the base of the wells is constituted by a filter and that said wells contain membrane fractions as defined in any one of claims 1 to 7.

## Patentansprüche

1. Plasmamembranfraktionen von prokaryontischen oder eukaryontischen tierischen Zellen, die wenigstens eine rekombinante Monogalaktosyldiacylglycerin (MGDG)-Synthase enthalten, **dadurch gekennzeichnet, dass** die Fraktionen wenigstens 1 Gew.-% 1,2-sn-Diacylglycerin (DAG) enthalten, bezogen auf das Gesamtproteingewicht, und dass sie in Form kugelförmiger Membranvesikel vorliegen, die aus einer Lipiddoppelschicht gebildet sind.

2. Fraktionen nach Anspruch 1, **dadurch gekennzeichnet, dass** das Molverhältnis von MGDG-Synthase/DAG weniger als 10 ist.

3. Fraktionen nach Anspruch 2, **dadurch gekennzeichnet, dass** das Molverhältnis von MGDG-Synthase/DAG weniger als 0,12 ist.

4. Fraktionen nach irgendeinem der vorhergehenden Ansprüche, **dadurch gekennzeichnet, dass** die Membranvesikel eine Größe zwischen 0,1 µm und 10 µm haben.

5. Fraktionen nach irgendeinem der vorhergehenden Ansprüche, **dadurch gekennzeichnet, dass** die MGDG-Synthase auf der Innenseite der Lipiddoppelschicht lokalisiert ist.

6. Fraktionen nach irgendeinem der vorhergehenden Ansprüche, **dadurch gekennzeichnet, dass** die Vesikel in Form nichtinvertierter, invertierter oder hybrider Vesikel vorliegen.

7. Fraktionen nach Anspruch 6, **dadurch gekennzeichnet, dass** sie in Form hybrider Vesikel vorliegen, die MGDG-Synthase und DAG in der gleichen Lipidschicht tragen.

8. Verfahren zur Herstellung von Membranfraktionen gemäß irgendeinem der vorhergehenden Ansprüche, **dadurch gekennzeichnet, dass** es besteht:
- in einem ersten Schritt, Transformieren der prokaryontischen oder eukaryontischen tierischen Zellen mit einem Konstrukt, das das Gen enthält, das für eine Pflanzen-MGDG-Synthase codiert,
- in einem zweiten Schritt, Kultivieren der Zellen in einem Kulturmedium, das die Proteinsynthese begünstigt, so dass die Synthese der MGDG-Synthase durch die Zellen induziert wird,
- in einem dritten Schritt, Inkubieren der in dem vorhergehenden Schritt kultivierten Zellen in einem Reaktionsmedium, das wenigstens eine Phospholipase C enthält,
- und dann in einem vierten Schritt, Fraktionieren der so an DAG angereicherten Zellen, um Membranfraktionen in Form kugelförmiger Vesikel zu erhalten, die aus einer Lipiddoppelschicht gebildet sind und wenigstens eine rekombinante MGDG-Synthase und wenigstens 1 Gew.-% DAG enthalten, bezogen auf das Gesamtproteingewicht der Membranfraktionen.

9. Verfahren nach Anspruch 8, **dadurch gekennzeichnet, dass** die prokaryontischen Zellen Bakterien sind.

10. Verfahren nach Anspruch 8 oder 9, **dadurch gekennzeichnet, dass** man eine Phospholipase C von *Bacillus cereus* verwendet.

11. Verfahren nach irgendeinem der Ansprüche 8 bis 10, **dadurch gekennzeichnet, dass** die Phospholipase C in einer Konzentration zwischen 1 U und 20 U pro ml Reaktionsmedium verwendet wird.

12. Verfahren nach irgendeinem der Ansprüche 8 bis 11, **dadurch gekennzeichnet, dass** die Fraktionierung der Membranen durch mechanischen Schock, Thermoschock, osmotischen Schock, elektrischen Schock oder physischen Schock erfolgt.

13. Verwendung der Membranfraktionen gemäß irgendeinem der Ansprüche 1 bis 7 zur Selektion und/oder zum Screenen *in vitro* von Effektormolekülen für die MGDG-Synthase-Aktivität.

14. Verwendung nach Anspruch 13 zur Selektion und/oder zum Screenen *in vitro* von Inhibitormolekülen für die MGDG-Synthase-Aktivität, die als Wirkstoffe gegen Parasiten oder als Herbizide dienen können.

15. Verfahren zur Selektion und/oder zum Screenen von Effektormolekülen für die MGDG-Synthase-Aktivität, **dadurch gekennzeichnet, dass** es umfasst:
- einen Inkubationsschritt der Testsubstanz oder der Testsubstanzen mit einer ausreichenden Menge Membranfraktionen gemäß irgendeinem der Ansprüche 1 bis 7 und mit radiomarkierter UDP-Galaktose in einer wässrigen Phase mit einem pH zwischen 4 und 11;
- einen Schritt mit ein oder mehrmaligem Waschen der Membranfraktionen,
- einen Schritt des Abtrennens der Membranfraktionen,
- und dann einen Schritt zur Bestimmung der Aktivität der MGDG-Synthase.

16. Verfahren nach Anspruch 15, **dadurch gekennzeichnet, dass** die wässrige Inkubationsphase einen Puffer enthält und einen pH zwischen 6 und 8 hat.

17. Verfahren nach Anspruch 15 oder 16, **dadurch gekennzeichnet, dass** die Menge an UDP-Galaktose zwischen 0,1 und 10 nmol pro µl Reaktionsmedium liegt.

18. Verfahren nach irgendeinem der Ansprüche 15 bis 17, **dadurch gekennzeichnet, dass** der Schritt des Abtrennens der Membranfraktionen mittels Zentrifugation oder Filtration durchgeführt wird.

19. Verfahren nach irgendeinem der Ansprüche 15 bis 18, **dadurch gekennzeichnet, dass** die Bestimmung der Aktivität der MGDG-Synthase durch Messung der Menge an radiomarkierter Galaktose erfolgt, die in die Membranfraktionen eingebaut ist.

20. Mikrotiterplatte umfassend eine Vielzahl von Vertiefungen, **dadurch gekennzeichnet, dass** der Boden der Vertiefungen aus einem Filter besteht und dass die Vertiefungen Membranfraktionen gemäß irgendeinem der Ansprüche 1 bis 7 enthalten.
